# EUROPEAN PATENT SPECIFICATION

(11) **EP 3 549 576 B1**
(45) Date of publication and mention of the grant of the patent: **07.06.2023**
(21) Application number: 19386015.2
(22) Date of filing: 22.03.2019
(51) Int. Cl.: A61K 9/08, A61K 47/26, A61K 31/519, A61K 33/26, A61K 9/14

(54) **A PHARMACEUTICAL COMPOSITION FOR THE TREATMENT OF ANEMIA IN A SUITABLE CONTAINER, WHICH KEEPS THE ACTIVE INGREDIENTS SEPARATED IN A SEALED MANNER: THE FERRIC COMPLEX WITH N-ACETYL-L-ASPARTIC CASEIN AND CALCIUM FOLINATE PENTAHYDRATE**
PHARMAZEUTISCHE ZUSAMMENSETZUNG ZUR BEHANDLUNG VON ANÄMIE IN EINEM GEEIGNETEN BEHÄLTER, DER WIRKSTOFFE ABGEDICHTET GETRENNT HÄLT:EISENKOMPLEX MIT N-ACETYL-L-ASPARAGIN-CASEIN UND CALCIUM-FOLINAT-PENTAHYDRAT
COMPOSITION PHARMACEUTIQUE POUR LE TRAITEMENT DE L'ANÉMIE DANS UN RÉCIPIENT ADAPTÉ, QUI CONSERVE LES INGRÉDIENTS ACTIFS SÉPARÉS SELON UNE MÉTHODE SCELLÉE :COMPLEXE FERRIQUE AVEC DE LA CASÉINE N-ACÉTYL-L-ASPARTIQUE ET FOLINATE PENTAHYDRATE DE CALCIUM

(30) Priority: 05.04.2018 GR 20180100151
(43) Date of publication of application: 09.10.2019
(73) Proprietor: Tseti, Ioulia, 145 61 Kifissia Attikis (GR)
(72) Inventor: Tseti, Ioulia, 145 61 Kifissia Attikis (GR)
(74) Representative: Wibbelmann, Jobst

(56) References cited:
- EP-A1- 0 427 078
- EP-A1- 2 922 767
- US-A- 4 931 441
- LAZZARI FLAVIO ET AL: "Overview of clinical trials in the treatment of iron deficiency with iron-acetyl-aspartylated casein", CLINICAL DRUG INVESTIGATION, ADIS INTERNATIONAL, AUCKLAND, NZ, vol. 25, no. 11, 1 January 2005 (2005-01-01), pages 679-689, XP009182971, ISSN: 1173-2563, DOI: 10.2165/00044011-200525110-00001

## Description

The present invention relates to a pharmaceutical composition in the form of a single dose oral solution, as an iron source, which comprises as active ingredients the ferric complex with N-acetyl-L-aspartic casein and calcium folinate pentahydrate. The ferric complex with N-acetyl-L-aspartic casein is standardized in trivalent iron and complexed N-acetyl-L-aspartic acid and stored as a solution in a suitable container. The calcium folinate pentahydrate is stored in powder form in a storage area in the container closure. The two active ingredients kept separated in a sealed manner and mixed immediately before use.

The ferric complex with N-acetyl-L-aspartic casein (ICA) is an amorphous, slightly hygroscopic powder that is insoluble in all organic solvents and is soluble only in alkaline aqueous solutions at pH 8. ICA complex is an Iron (III) complex with N-acetyl-L-aspartic derivative of casein. ICA complex acts as an effective carrier for iron presenting a positive and well-tolerable safety profile. The main feature of ICA complex is that it restricts the interaction of iron ions with the gastric mucosa, thus preventing the formation of high molecular weight iron hydroxides, which are poorly absorbed. Then, ICA complex is hydrolyzed by pancreatic proteases at the alkaline pH of the duodenum and iron (III) in bioavailable form is absorbed in the small intestine. In this context, most of the adverse effects associated with iron (II) sulphate administration, namely, the low absorption capacity and the higher incidence of adverse effects due to long-term treatment, are avoided. Studies related to dosage unit response revealed that the administration of 80 mg of ICA is able to result in an extremely rapid increase in serum iron levels. The increase of iron levels in serum due to ICA complex administration observed in an earlier stage and lasted longer, compared to ferritin administration. Thus, ICA complex provides a rapid and sustained source of bioavailable iron at doses equal to those of similar products. Furthermore, multiple dosing administration of ICA complex increase iron levels in serum in an indistinguable manner compared to a single dose administration. Thus, ICA complex is a suitable candidate for long-term or repeated therapy. In this context, excellent tolerability observed in ICA administration, even with therapeutic regimens lasting up to 5 months.

*Lazzari et al.* provide an overview of the pharmacokinetic and clinical data on ICA complex in paediatric and adult patients affected by iron deficiency anaemia.

Folinic acid is the 5-formyl derivative of tetrahydrofolic acid, an intermediate product of folate metabolism and represents its active biological form. Folic acid is metabolized within the body, to a tetrahydrofolate derivative, which is a co-enzyme responsible for several metabolic processes, including the purine and pyrimidine nucleotide synthesis and DNA synthesis in the hematopoietic system. Folinic acid administration contributes to the treatment of vitamin B deficiency, even in the absence of hepatic enzymes that activate the conversion of folic to folinic acid. Folic acid has excellent anti-anemic action and is effective in anemia due to lack of folic acid. Calcium folinate is easily soluble in aqueous solutions, can be absorbed by the proximal part of the small intestine and is rapidly distributed to the tissues, where it is converted into biologically active folinic acid. The excess of calcium folinate is excreted in urine.

Clinical studies have demonstrated the beneficial effect of ICA complex in the increase of iron levels in the blood. Other studies performed in pediatric patients showed that administration of ICA complex was well tolerated and no undesirable effects were observed, in contrast to patients treated with iron succinylated protein, where severe gastrointestinal reactions (nausea, vomiting and diarrhea) were observed requiring discontinuation of therapy.

The pharmaceutical composition of the invention addresses the problem of the undesirable side effects observed from the use of iron succinylated protein complexes using the ferric complex with N-acetyl-L-aspartic casein, which exhibits better bioavailability and much less side effects. The pharmaceutical composition is further enriched in calcium folinate pentahydrate, a precursor of folinic acid which presents a beneficial effect on iron absorption, especially during pregnancy and lactation.

The present invention relates to a pharmaceutical composition suitable for the prevention of anemia, comprising as active components the ferric complex with N-acetyl L-aspartic casein and calcium folinate pentahydrate, which are stored separately in a sealed manner in a suitable container and are mixed at the time of use. The container consists of a cylindrical vial suitable to store a liquid composition, a closure cap with an internal storage space, suitable to store a solid composition and suitable elements which ensure the separation in a sealed manner of the solid composition contained in the closure cap from the liquid composition contained in the cylindrical vial during storage, while, they allow the two compositions to get mixed by a single movement at the time of use.

A preferable, non-binding but illustrative container used in the present invention is the one described in EP2922767B1 (INGE S.p.A.). The container consists of a cylindrical vial and a cylindrical closure cap, comprising an edge portion, which is adapted to seal the mouth of the container, preferably a bottle, for example by means of an inner thread. In this way, the mouth of the container is protected against possible bacterial contaminations. The closure cap has, on the upper side, a deformable portion and a hollow space in the shape of the closure cap and on the lower side, a conical portion which can be inserted, by applying pressure, into the mouth of the container. The deformable conical portion which has the shape of the closure cap, is closed on the lower side by a thermowelded film. In this way, a chamber suitable to house an active substance in solid form is defined, said solid active substance is separate in a sealed manner from the other active substance which is housed in the cylindrical bottle in liquid form. The chamber is obviously filled before being closed by welding the film. The conical portion of the closure cap, further comprising a series of breaking elements on the lower side which are capable to break the welded film by applying pressure to the upper deformable portion of the closure cap. As a consequence, the first substance housed in the closure cap in solid form falls into the container and get mixed with the solution of the other active substance housed in the bottle. Then, the user can break the sealing means and open the container by removing the closure cap, thus using the mixture. Similar containers under the same philosophy are well known in a skilled person and can be used in the present invention.

In a preferred embodiment, the vials of the container of the invention contain 15 mL of a solution of the ferric complex with N-acetyl L-aspartic casein, while the cylindrical closure cap with the sealed chamber contains 200 mg of calcium folinate pentahydrate in powder form.

In a preferred embodiment, each vial containing 15 mL of a solution of the ferric complex with N-acetyl L-aspartic casein, said vial being sealed with the cylindrical closure cap with the storage space containing calcium folinate pentahydrate in solid form, refers to a single dosage unit for immediate delivery of the pharmaceutical composition of the invention.

The ferric complex with N-acetyl L-aspartic casein utilized in the present invention is prepared following the method described in EP3034512 B2 and presents the characteristics of Table 1.

**Table 1. Specifications of ferric complex with N-acetyl L-aspartic casein**

| Technical Parameters | Specifications |
|---|---|
| Color | Brown-red |
| Solubility | Soluble in alkaline aqueous solutions pH = 8 |
| Loss on Drying | < 10 % w/w |
| pH of a 5% in Fe³⁺ suspension | 2.5 - 3.5 |
| Free Iron | < 0.05 % w/w |
| Total Fe³⁺ | 4.7 - 5.3 % w/w |
| Proteins | > 72 % w/w |
| Complexed N-Acetyl-L-Aspartic Acid | 7 - 10 % w/w |
| Free N-Acetyl-L-Aspartic Acid | < 2 % |
| Free Chlorides | < 3 % |
| TAMC | < 1000 cfu/g |
| TYMC | < 100 cfu/g |
| E. coli | Absent in 1.0 gr of the complex |

In a preferred embodiment, the quantity of the ferric complex with N-acetyl L-aspartic casein in the pharmaceutical composition is in the range of 600 to 1000 mg per dosage unit, and corresponds from 30 to 50 mg of trivalent iron per dosage unit, more preferably is 800 mg and corresponds to 40 mg trivalent iron per dosage unit.

In a preferred embodiment, the quantity of calcium folinate pentahydrate in the pharmaceutical composition is in the range of 0.175 mg to 0.312 mg per dosage unit and corresponds from 0.138 mg to 0.246 mg folinic acid, more preferably is 0.235 mg and corresponds to 0.185 folinic acid per dosage unit.

### Example 1: Preparation and conservation of the active substance in liquid form.

Propyleneglycol and sorbitol were mixed in purified water under stirring till complete homogenization. Subsequently, p-hydroxybenzoic methylester and *p*-hydroxybenzoic propylester were added to the above solution and the pH is adjusted to 8.0 ± 0.2 in the aid of a 0.1 N NaOH solution. Then, the ferric complex with N-acetyl L-aspartic casein was added in portions, while the pH of the solution was maintained at 8.0 ± 0.2 in the aid of a 1 N NaOH solution. After the complete solubilization of the ferric complex with N-acetyl L-aspartic casein, the solution was further stirred for 8 hours. Finally, the flavoring agent was added to the brown-red solution and after gentle agitation for 2 hours, the solution was filtered through a 150 µm filter to remove any insoluble material. The cylindrical container was filled with the filtered clear solution which then was sealed with the closure cap containing the hollow space.

### Example 2: Preparation and conservation of the active substance in solid form

Calcium folinate pentahydrate and mannitol were placed in a suitable drum-type mixer and homogenized for thirty minutes at 45 rpm. The final blend after homogenization procedure was checked for uniformity, placed in the closure caps, which then were sealed air tight.

The final pharmaceutical preparation comprises the following ingredients

| **Ingredients in liquid form (Final Volume 15 mL)** | **Quantity (mg)** |
|---|---|
| Ferric complex with N-acetyl L-aspartic casein | 800 (corresponds to 40 mg Fe³⁺) |
| Sorbitol | 1400 |
| Propyleneglycol | 1000 |
| p-hydroxybenzoic methylester | 45 |
| *p*-hydroxybenzoic propylester | 15 |
| Toffee | 150 |
| Purified Water | q.s. 15 mL |
| **Ingredients in powder form (Final Quantity 200 mg)** | |
| Calcium folinate pentahydrate | 0.235 (corresponds to 0.185 mg folinic acid) |
| Mannitol | up to 200 mg |

### Literature

EP 3 034 512 B2 A process for producing iron (III) casein N-acetyl-aspartylated complexes and use thereof in pharmaceutical compositions
EP 2 922 767 B1 Device for the fluid-tight conservation of a substance to be mixed to another substance contained in a container

Lazzari Flavio et al.: "Overview of clinical trials in the treatment of iron deficiency with iron-acetyl-aspartylated casein", CLINICAL DRUG INVESTIGATION, ADIS INTERNATIONAL, AUCKLAND, NZ, vol. 25, no. 11, 1 January 2005, pages 679-689

## Claims

1. A pharmaceutical composition stored in a sealed manner in a container comprising a cylindrical bottle and a closure storage cap, said pharmaceutical composition comprising as active ingredients ferric complex with N-acetyl-L-aspartic casein and calcium folinate pentahydrate, the two active ingredients to be mixed together creating a single dose oral solution at the time of use, wherein the ferric complex with N-acetyl-L-aspartic casein is stored in liquid form in the cylindrical bottle and calcium folinate pentahydrate is stored in solid form in the hollow space of the closure storage cap which seals the bottle.

2. The pharmaceutical composition according to claim 1 wherein the quantity of ferric complex with N-acetyl-L-aspartic casein is 800 mg per dosage unit of oral solution and the quantity of calcium folinate pentahydrate is 0.235 mg per dosage unit of oral solution.

3. The pharmaceutical composition according to claims 1 and 2 wherein the ferric complex with N-acetyl-L-aspartic casein in the oral solution is standardized in 4.7-5.3 % w/w in trivalent iron and in 7-10 % w/w in complexed N-Acetyl-L-aspartic acid.

## Patentansprüche

1. Pharmazeutische Zusammensetzung, die in versiegelter Weise in einem Behälter aufbewahrt wird, der eine zylindrische Flasche und eine Verschlusskappe zur Aufbewahrung umfasst, wobei die pharmazeutische Zusammensetzung als aktive Bestandteile Eisen(III)-Komplex mit N-Acetyl-L-Asparagin-Casein und Calciumfolinat-Pentahydrat umfasst, wobei die beiden Wirkstoffe zusammengemischt werden sollen, um eine orale Einzeldosislösung zum Zeitpunkt der Verwendung zu erzeugen, wobei der Eisen(III)-Komplex mit N-Acetyl-L-Aspartat-Casein in flüssiger Form in der zylindrischen Flasche gelagert wird und Calciumfolinat-Pentahydrat in fester Form in dem Hohlraum der Verschlussaufbewahrungskappe gelagert wird, die die Flasche abdichtet.

2. Pharmazeutische Zusammensetzung nach Anspruch 1, wobei die Menge an Eisen(III)-Komplex mit N-Acetyl-L-Asparaginsäure-Casein 800 mg pro Dosierungseinheit der oralen Lösung beträgt und die Menge an Calciumfolinat-Pentahydrat 0,235 mg pro Dosierungseinheit der oralen Lösung beträgt.

3. Pharmazeutische Zusammensetzung nach Anspruch 1 und 2, wobei der Eisen(III)-Komplex mit N-Acetyl-L-Asparaginsäure-Casein in der oralen Lösung auf 4,7-5,3 Gew.-% dreiwertiges Eisen und auf 7-10 Gew.-% komplexierte N-Acetyl-L-Asparaginsäure standardisiert ist.

## Revendications

1. Composition pharmaceutique stockée de manière étanche dans un récipient comprenant un flacon cylindrique et un bouchon de fermeture et conservation, ladite composition pharmaceutique comprenant comme principes actifs un complexe ferrique avec de la caséine N-acétyl-L-aspartique et du folinate de calcium pentahydraté, les deux principes actifs à mélanger ensemble créant une solution buvable unidose au moment de l'utilisation, dans laquelle le complexe ferrique avec la caséine N-acétyl-L-aspartique est stocké sous forme liquide dans le flacon cylindrique et le folinate de calcium pentahydraté est stocké sous forme solide dans l'espace creux du bouchon de fermeture et conservation qui scelle le flacon.

2. Composition pharmaceutique selon la revendication 1, dans laquelle la quantité de complexe ferrique avec la caséine N-acétyl-L-aspartique est de 800 mg par dose unitaire de solution buvable et la quantité de folinate de calcium pentahydraté est de 0,235 mg par dose unitaire de solution buvable.

3. Composition pharmaceutique selon les revendications 1 et 2, dans laquelle le complexe ferrique avec la caséine N-acétyl-L-aspartique dans la solution buvable est standardisé entre 4,7 et 5,3 % p/p en fer trivalent et entre 7 et 10 % p/p en acide N-acétyl-L-aspartique complexé.
